# EUROPÄISCHE PATENTSCHRIFT

(11) **EP 1 710 224 B1**
(45) Veröffentlichungstag und Bekanntmachung des Hinweises auf die Patenterteilung: **19.12.2007**
(21) Anmeldenummer: 06112093.7
(22) Anmeldetag: 31.03.2006
(51) Int. Cl.: C07C 45/40, C07C 49/21

(54) **Verfahren zur Darstellung von Ketonen durch Ozonolyse**
Process for the preparation of ketones by ozonolysis
Procédé pour la préparation de cétones par ozonolyse

(30) Priorität: 05.04.2005 DE 102005015590
(43) Veröffentlichungstag der Anmeldung: 11.10.2006
(73) Patentinhaber: Symrise GmbH & Co. KG, 37603 Holzminden (DE)
(72) Erfinder: Nobis, Markus, 3250 Lyss (CH)
(74) Vertreter: Eisenführ, Speiser & Partner

(56) Entgegenhaltungen:
- EP-A- 0 822 191
- EP-A- 1 215 190

## Beschreibung

Die Erfindung betrifft ein Verfahren zur Darstellung eines Ketons aus einem Alkohol mit einer alpha-ständigen Doppelbindung.

Alkohole mit einer alpha-ständigen Doppelbindung werden herkömmlicherweise in Gegenwart anorganischer Oxidationsmittel (z. B. KMnO₄, OsO₄, H₂SO₄/H₂CrO₄) in Ketone überführt. Insbesondere ist bekannt, einen Alkohol der Formel (I) auf diese Weise in ein Keton der Formel (II) zu überführen: 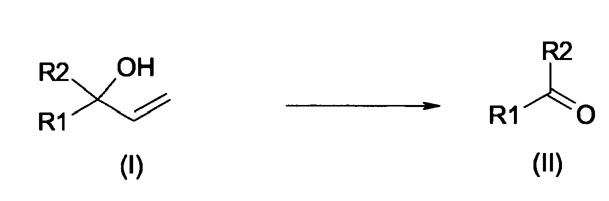 wobei die Reste R1, R2 unabhängig voneinander Alkyl, Alkenyl, Cycloalkyl oder Aryl sein können. Alkohole der Formel (I) werden teilweise aus natürlichen Quellen bezogen, sind jedoch auch auf synthetischem Wege zugänglich.

WO 91/09852 beschreibt ein zweistufiges Verfahren zur Herstellung von Sclareolid (auch (-)-Norlabdanoxid) aus Sclareol, bei dem in erster Stufe ein oxidativer Abbau von Sclareol in Gegenwart von Rutheniumsalzen oder Kaliumpermanganat und in einer zweiten Stufe das gebildete Zwischenprodukt mit der Persäure und/oder Persäuresalzen zum Sclareolid oxidiert wird.

Barton et al. (Tetrahedron Letters, 1994, 35(32), 5801) beschreiben einen weiteren synthetischen Ansatz zur Darstellung von Sclareolid ausgehend von Sclareol und zwar durch oxidative Umsetzung des Ausgangsproduktes mit einem Gemisch aus OsO₄/NalO₄.

Die in herkömmlichen Verfahren genutzten Oxidationsmittel sind aufgrund ihrer Giftigkeit für Mensch und Umwelt sowie ihrer dadurch erschwerten Handhabbarkeit nachteilig. Dieser Nachteil erschwert insbesondere die industrielle Umsetzung von Alkoholen mit einer alpha-ständigen Doppelbindung.

Es ist deshalb versucht worden, diese Verfahren abzuändern und insbesondere neue Oxidationsmittel zu verwenden. So beschreiben die EP 0 822 191 A1 und Fekih et al. (J. Soc. Chim. Tunisie, 2001, 4(9), 909) jeweils zweistufige Verfahren zur Darstellung von Sclareoloxid aus Sclareol durch Ozonolyse: 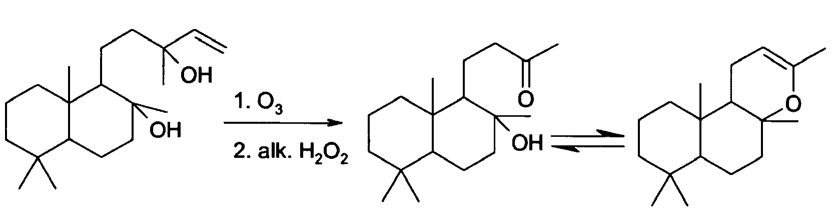 In einer ersten Stufe wird die Allylalkohol-Gruppe des Sclareols durch Addition von Ozon zum entsprechenden Ozonid umgesetzt. In einer zweiten Stufe wird anschließend das Ozonid durch Aufarbeitung mit alkalischem H₂O₂ in das gewünschte Sclareoloxid überführt. Die Reaktion kann in verschiedenen organischen Lösungsmitteln, wie Dichlormethan, Methanol oder Ethanol durchgeführt werden. Bei der Umsetzung fallen jedoch im ersten Schritt große Mengen des stark reaktiven Ozonids an, so dass erhebliche Sicherheitsmaßnahmen für das Durchführen der Reaktion erforderlich sind. Insbesondere wird eine leistungsfähige Kühlung benötigt, um die Reaktion sicher durchführen zu können. Diese Nachteile fallen besonders bei einer industriellen Umsetzung stark ins Gewicht.

Der Erfindung liegt die Aufgabe zugrunde, ein Verfahren zum Darstellen eines Ketons aus einem tertiären Alkohol mit einer alpha-ständigen Doppelbindung anzugeben, das die oben angegebenen Nachteile herkömmlicher Verfahren begrenzt oder ganz vermeidet. Das Verfahren sollte insbesondere ohne die bisher erforderlichen starken Sicherheitsmaßnahmen durchführbar sein.

Die Aufgabe wird gelöst durch ein Verfahren zur Darstellung eines Ketons aus einem tertiären Alkohol mit einer alpha-ständigen Doppelbindung, umfassend die Schritte:
a) Bereitstellen des Alkohols,
b) Behandeln des Alkohols mit Ozon in Anwesenheit einer anorganischen Base.

Das erfindungsgemäße Verfahren führt zu einer überraschend kurzen Reaktionszeit bei gleichzeitig hoher Ausbeute und vermeidet das zeitweise Auftreten großer Wärmemengen. Zudem wird auf den Einsatz stark giftiger Oxidationsmittel verzichtet, so dass insgesamt geringere Sicherheitsmaßnahmen getroffen werden müssen als bei herkömmlichen Verfahren.

Die Erreichbarkeit hoher Ausbeuten bei gleichzeitig kurzer Reaktionszeit war insbesondere deshalb überraschend, da bekannt war, dass Ozon in Anwesenheit einer anorganischen Base insbesondere bei schwächer basischen pH-Werten rasch zerfällt (Hollemann, Wiberg, Lehrbuch der Anorganischen Chemie, 101. Aufl., S. 516). Dementsprechend war zu erwarten, dass große Mengen an Ozon benötigt würden, um eine ausreichende Menge an Ozon zum Umsetzen des Alkohols bereitzustellen. Es hat sich nunmehr überraschenderweise herausgestellt, dass die Menge an benötigtem Ozon trotz der Anwesenheit einer anorganischen Base im Vergleich zu herkömmlichen Verfahren nicht erhöht ist, und dass die erfindungsgemäße, in einem Schritt durchgeführte Reaktion sogar deutlich schneller und mit geringerem Bedarf an Sicherheitsmaßnahmen als bei herkömmlichen Verfahren durchführbar ist.

Ein weiterer Vorteil des erfindungsgemäßen Verfahrens ist, dass in Schritt b) nur geringe Wärmemengen freigesetzt werden. Im Vergleich zu herkömmlichen Verfahren werden deshalb zum Durchführen des erfindungsgemäßen Verfahrens nur noch Kühlaggregate mit geringerer Leistung benötigt. Dies ist insbesondere bei einer industriellen Verfahrensführung ein großer Vorteil.

Besonders gute Ergebnisse können erhalten werden, wenn in Schritt b) der eingesetzte Alkohol mit 1-3 Moläquivalenten Ozon, bezogen auf die umzusetzende Alkohol-Gruppe, behandelt wird. Besonders bevorzugt ist dabei ein erfindungsgemäßes Verfahren, bei dem in Schritt b) der eingesetzte Alkohol mit 1-2 Moläquivalenten Ozon, bezogen auf die umzusetzende Alkohol-Gruppe, behandelt wird. In beiden Fällen wird die Menge an eingesetztem Ozon vorteilhaft gering gehalten. Dies ist insbesondere bei einer industriellen Verfahrensdurchführung von Vorteil, da Ozon zweckmäßigerweise während der Umsetzung des Alkohols in einer parallel laufenden Reaktion erzeugt und fortgesetzt oder kontinuierlich der in Schritt b) ablaufenden Reaktion zugesetzt wird. Das erfindungsgemäße Verfahren ermöglicht daher eine Umsetzung des Alkohols bei einem geringen Bedarf an bereitzustellendem Ozon.

Zur Erzeugung des Ozons in einem Ozongenerator können reiner Sauerstoff aber auch Gemische aus Sauerstoff und inerten Gasen in verschiedenen Volumen-Verhältnissen an Sauerstoff, vorzugsweise zwischen 1 und 80 Vol.%, verwendet werden. Ein Ozongehalt eines in Schritt b) in das Reaktionsgemisch eingeleiteten Gases liegt vorzugsweise im Bereich von 1 bis 12 Gew.% bezogen auf das eingesetzte Gas, besonders bevorzugt aber im Bereich von 4 bis 8 Gew.%. Das Ozon kann in einer molaren Menge im Bereich von 1 bis 5, bevorzugt im Bereich von 1 bis 3, besonders bevorzugt im Bereich von 1,1 bis 2 Mol-Äquivalenten zur umzusetzenden alpha-ständigen Doppelbindung der Verbindung in das Reaktionsgemisch eingeleitet werden. Hierdurch lassen sich Nebenprodukte der Ozonolyse mindern.

Ferner ist es bevorzugt, wenn in Schritt b) die Base nicht bereits zu Beginn der Reaktion vollständig vorgelegt wird, sondern so fortgesetzt zugesetzt wird, dass ihre Äquivalentkonzentration bei Abbruch der Reaktion bezogen auf die insgesamt eingesetzten, umzusetzenden Alkoholgruppen 1 bis 3, vorzugsweise 1 bis 2 beträgt. Auf diese Weise ist sichergestellt, dass die Konzentration an verfügbarem Ozon optimal hoch ist, um eine rasche Umsetzung des Alkohols unter hohen Ausbeuten zu erzielen, und gleichzeitig gering genug ist, um das Freisetzen hoher Wärmemengen in Schritt b) zu verhindern. Die Base kann bei Durchführen des Schrittes b) kontinuierlich oder wiederholt zugesetzt werden.

Als anorganische Basen eignen sich alle unter Ozonolysebedingungen stabilen starken bis mittelstarken Brönstedtbasen. Der pKB-Wert der Base beträgt vorzugsweise 4 bis 10. Die in Schritt b) verwendete Base ist vorzugsweise ausgewählt aus der Gruppe bestehend aus NaOH, KOH, LiOH, NaHCO₃ Na₂CO₃, CaCO₃ oder Mischungen zweier oder mehrerer dieser Basen. Vorteilhaft wiederum sind dabei die genannten Alkalimetallbasen, bevorzugt sind die Alkalimetallhydroxide. Besonders bevorzugte Basen sind LiOH, NaOH und KOH. Mit Alkalimetallbasen werden in dem erfindungsgemäßen Verfahren keine entsprechenden Alkalimetallperoxide oder Alkalimetallozonide gebildet bzw. akkumuliert, im Gegensatz zu dem in US 3,664,810 beschriebenen Verfahren mit Erdalkalimetallbasen, bei dem im Wesentlichen stöchiometrische Mengen der entsprechenden Erdalkaliperoxide entstehen. Diese Basen, insbesondere die (besonders bevorzugt genannten) Alkalimetallhydroxide, haben in Vergleichsversuchen besonders hohe Ausbeuten der gewünschten Verbindung geliefert. Die Basen werden in Schritt b) zweckmäßigerweise in gelöster Form bereitgestellt, so dass bei der Auswahl der Base auch deren Löslichkeit in dem verwendeten Lösungsmittel zu berücksichtigen ist.

Die EP 1 569 885 betrifft die in-situ Zersetzung von Peroxiden bei der Ozonolyse von gegebenenfalls substituierten Alkenen zu den korrespondierenden Aldehyden oder Ketonen. Als Trägermaterial für die dort verwendeten Peroxid-zersetzenden Metallkatalysatoren kann unter anderem CaCO₃ verwendet werden. Gemäß EP 1 569 885 wird CaCO₃ nicht wegen seiner basischen Eigenschaften eingesetzt, sondern im Gegenteil als inertes Trägermaterial, welches sich in dem bei der Ozonolyse eingesetzten Lösungsmittel(gemisch) nicht auflöst und daher auch keinen nennenswerten Beitrag zum pH-Wert liefert.

Vorzugsweise wird das erfindungsgemäße Verfahren bei einem pH-Wert im Bereich von 13 bis 8 durchgeführt. Regelmäßig liegt der pH-Wert während der Ozonolyse in Anwesenheit der Base in Schritt b) des erfindungsgemäßen Verfahrens anfangs im Bereich von 13 bis 12, zum Ende der Ozonolyse im Bereich von 9 bis 8. Dabei ist anzumerken, dass die Selektivität der Ozonolyse in dem erfindungsgemäßen Verfahren mit fortschreitender Reaktionsdauer im Wesentlichen nicht abnimmt. Dies ist umso überraschender als bekannt ist, dass die Stabilität des Ozons bei niedrigeren pH-Werten (unterhalb von 14) merklich abnimmt (Hollemann, Wiberg, Lehrbuch der Anorganischen Chemie, 101. Aufl., S. 508, 517). Es wäre zu erwarten gewesen, dass die Zerfallsprodukte des Ozons mit den erfindungsgemäß einzusetzenden tertiären Alkoholen mit einer alpha-ständigen Doppelbindung zu unerwünschten Neben- oder Abbauprodukten der tertiären Alkohole reagiert hätten.

In bevorzugten Ausgestaltungen wird das erfindungsgemäße Verfahren in Abwesenheit eines heterogenen, anorganischen Peroxid-zersetzenden Katalysators aus der Gruppe Iridium, Mangan, Kobalt, Silber, Gold, Palladium, Platin oder Ruthenium durchgeführt.

In weiteren bevorzugten Ausgestaltungen wird das erfindungsgemäße Verfahren in Abwesenheit eines Emulgators durchgeführt.

Vorzugsweise wird als Lösungsmittel für die Base in Schritt b) Wasser oder ein Lösungsmittelgemisch aus Wasser und einem mit Wasser mischbaren organischen Lösungsmittel eingesetzt. Das Lösungsmittelgemisch besteht vorzugsweise aus Tetrahydrofuran und Wasser, insbesondere mit einem Massenmischungsverhältnis von Tetrahydrofuran zu Wasser im Bereich von 1:2 bis 2:1, besonders bevorzugt etwa 1:1. Das Lösungsmittel muss für die Ozonolyse geeignet sein. Die Base wird vorzugsweise in Schritt b) durch Zutropfen aus einer Stammlösung zugesetzt, wobei die Konzentration der Base in der Stammlösung vorzugsweise 2 bis 50 Gew.%, besonders bevorzugt 7,5 bis 10 Gew.%, jeweils bezogen auf die gesamte Stammlösung beträgt.

Das Lösungsmittel für den Alkohol ist so ausgewählt, dass es sich gegenüber Ozon vollständig oder weitgehend inert verhält und gegenüber der zugesetzten Base vollständig oder weitgehend stabil ist. Bevorzugte Lösungsmittel für den Alkohol umfassen substituierte oder nicht-substituierte aromatische Kohlenwasserstoffe, oder Lösungsmittel, die Sauerstoff in Form von Carbonyl-, Ether- oder Alkoholfunktionalitäten besitzen. Halogenierte aromatische und nichtaromatische Lösungsmittel erweisen sich ebenfalls als geeignet für die Durchführung der Reaktion. Lösungsmittel mit anderen oxidierbaren Heteroatomen (Stickstoff und Schwefel) sind aufgrund ihrer Affinität zu Sauerstoff nicht geeignet. Besonders bevorzugt ist Toluol.

Insbesondere ist bevorzugt, dass die Reaktion in Schritt b) in einem Zweiphasensystem durchgeführt wird, wobei in Schritt a) der Alkohol in einem organischen Lösungsmittel bereitgestellt wird und in Schritt b) die Base in einem wässrigen Lösungsmittel eingesetzt wird. Dies hat den Vorteil, dass eine Ausfällung der Base bei der Zugabe in das Reaktionsgemisch verhindert wird, eine Konzentration der Base in der den Alkohol enthaltenden Phase des Reaktionsgemisches gering bleibt und eine Reaktion zwischen dem gebildeten Ozonid und der Base nur im Bereich der Phasengrenzen erfolgt. Zweckmäßigerweise wird dabei das Reaktionsgemisch durch Rühren durchmischt. Besonders bevorzugt ist das Lösungsmittel des Alkohols Toluol und das Lösungsmittel der Base Wasser oder ein Lösungsmittelgemisch aus Wasser und Tetrahydrofuran, insbesondere mit einem Massenmischungsverhältnis von Tetrahydrofuran zu Wasser im Bereich von 1:2 bis 2:1, besonders bevorzugt etwa 1:1.

Vorzugsweise wird die Base in Schritt b) dem Reaktionsgemisch mit einer Zugaberate in Abhängigkeit von der Menge des gebildeten Ozonids zugesetzt. In der Regel wird die Zugaberate der Base erhöht, wenn sich auch die gebildete Menge des Ozonids im betrachteten Zeitraum erhöht, und umgekehrt. Besonders bevorzugt ist, wenn die gelöste Base in einer molaren Menge zwischen 0,8 bis 1,2 Mol-Äquivalenten zum gebildeten Ozonid zugesetzt wird. Hierdurch kann erreicht werden, dass die Konzentration des Ozonids im Reaktionsgemisch gering gehalten wird, aber mögliche Nebenreaktionen oder Störungen bei der Bildung des Ozonids aufgrund der zugesetzten Base vermieden werden.

Vorzugsweise beträgt in Schritt b) die Reaktionstemperatur -78 °C bis +30 °C, insbesondere -30°C bis +10°C, besonders bevorzugt -10°C bis 0°C. Hierdurch können Nebenreaktionen der Ozonolyse und bei der weiteren Umsetzung des gebildeten Ozonids mit der Base unterdrückt werden, aber gleichzeitig noch hinreichend hohe Umsätze für die beiden Teilreaktionen aufrecht erhalten werden.

Der in einem erfindungsgemäßen Verfahren, insbesondere nach einer der oben beschriebenen bevorzugten Verfahrensgestaltungen, eingesetzte Alkohol besitzt vorzugsweise die allgemeine Formel (la) 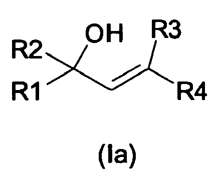 wobei R1 und R2 unabhängig voneinander einen organischen Rest bedeuten und beide Reste R1 und R2 gemeinsam einen Ring bilden können, und wobei ferner R3 und R4, unabhängig voneinander, Wasserstoff oder substituiertes oder nicht substituiertes Alkyl, Alkenyl, Cycloalkyl oder Aryl bedeuten können und beide Reste gemeinsam einen Ring bilden können und/oder einer oder beide Reste R3 und R4 mit einem oder beiden Resten R1 und/oder R2 gemeinsam einen Ring bilden können.

Das erfindungsgemäße Verfahren ist also mit einer vorteilhaft großen Auswahl an tertiären Alkoholen durchführbar.

In bevorzugten Alkoholen sind R1 und R2, unabhängig voneinander ausgewählt aus organischen Resten mit bis zu 30 Kohlenstoffatomen und bis zu 10 Stickstoff- und/oder Sauerstoffatomen.

Bevorzugt bedeuten R1 und R2 unabhängig voneinander, substituiertes oder nicht substituiertes Alkyl, Alkenyl, Cycloalkyl, Cycloalkylalkyl, Heterocycloalkyl oder (Hetero)Aryl, wobei R1 und R2 gemeinsam einen Ring, vorzugsweise einen insgesamt 5 bis 20 gliedrigen Ring, bilden können.

Besonders bevorzugt bedeuten R1 und R2, unabhängig voneinander, substituiertes oder nicht substituiertes Alkyl, Alkenyl, Cycloalkyl, Cycloalkylalkyl oder Aryl.

Bevorzugt bedeuten R3 und R4, unabhängig voneinander, Wasserstoff oder substituiertes oder nicht substituiertes Alkyl, Alkenyl, Cycloalkyl, Cycloalkylalkyl, Heterocycloalkyl oder (Hetero)Aryl, wobei R3 und R4 gemeinsam einen Ring, vorzugsweise einen insgesamt 5 bis 20 gliedrigen Ring, bilden können.

Sofern es sich bei R1, R2, R3 und/oder R4 um cyclische Reste handelt, sind hierunter auch miteinander verbundene Ringe, wie z.B. annelierte, polycyclische oder kondensierte Ringe, zu verstehen.

R1 und R2 bedeuten weiter bevorzugt unabhängig voneinander substituiertes oder nicht substituiertes gerad- oder verzweigtkettiges C₁-C₂₀-Alkyl, gerad- oder verzweigtkettiges C₃-C₂₀-Alkenyl, C₃-C₂₀-Cycloalkyl, C₄-C₂₀-Cycloalkylalkyl, C₃₋C₂₀-Heterocycloalkyl oder C₅-C₂₀-(Hetero)Aryl.

R3 und R4 bedeuten weiter bevorzugt unabhängig voneinander Wasserstoff oder substituiertes oder nicht substituiertes gerad- oder verzweigtkettiges C₁-C₂₀-Alkyl, gerad- oder verzweigtkettiges C₃-C₂₀-Alkenyl, C₃-C₂₀-Cycloalkyl, C₄-C₂₀₋Cycloalkylalkyl, C₃-C₂₀-Heterocycloalkyl oder C₅-C₂₀-(Hetero)Aryl.

Sofern es sich bei R1, R2, R3 und/oder R4 um substituiertes Alkyl, Alkenyl, Cycloalkyl, Cycloalkylalkyl, Heterocycloalkyl oder (Hetero)Aryl handelt, sind jeweils folgende Substituenten bevorzugt:
Hydroxy,
C₁-C₈-Alkyl, vorzugsweise Methyl, Ethyl, n-Propyl, iso-Propyl, n-Butyl, iso- Butyl, tert.- Butyl,
C₃-C₁₈-Cycloalkyl, vorzugsweise Cyclopropyl, Cyclopentyl, Cyclohexyl, Cyclooctyl, Cyclododecyl, Cyclopentadecyl, Cyclohexadecyl,
C₂-C₈-Alkinyl, vorzugsweise Ethinyl, Propinyl,
C₁-C₈-Perfluoralkyl, vorzugsweise Trifluormethyl,
C₁-C₄-Alkoxy, vorzugsweise Methoxy, Ethoxy, iso-Propoxy, n-Butoxy, iso-Butoxy, tert.-Butoxy,
C₃-C₁₂-Cycloalkoxy, vorzugsweise C₃-Cycloalkoxy, C₅-Cycloalkoxy, C₆₋Cycloalkoxy, C₈-Cycloalkoxy, C₁₂-Cycloalkoxy, C₁₅-Cycloalkoxy, C₁₆-Cycloalkoxy,
C₁-C₂₀-Alkoxyalkyl, in der 1 bis 5 CH₂-Gruppen durch Sauerstoff ersetzt sind, vorzugsweise -[-O-CH₂-CH₂-]ₙ-Q oder -[-O-CH₂-CHMe-]ₙ-Q, wobei Q = OH oder CH₃ ist und wobei n = 1 bis 4 bedeuten kann,
C₁-C₄-Acyl, vorzugsweise Acetyl,
C₁-C₄-Carboxy, vorzugsweise CO₂Me, CO₂Et, CO₂i-Pr, CO₂^{t}Bu,
C₁-C₄-Acyloxy, vorzugsweise Acetyloxy,
Halogenid, vorzugsweise F oder Cl, und
Si₁-Si₃₀-SilOXY-

Gute Ergebnisse werden insbesondere dann erhalten, wenn die alpha-ständige Doppelbindung nicht Teil eines Systems konjugierter Doppelbindungen ist. Vorzugsweise sind die Reste R3 und R4 deshalb Wasserstoff oder Alkyl. Besonders bevorzugt sind dabei Alkohole, bei denen R3 und R4 Wasserstoff sind.

Wenn der umzusetzende Alkohol neben einer umzusetzenden Alkohol-Gruppe weitere Alkohol-Gruppen oder andere Gruppen trägt, die nicht umgesetzt werden sollen, dann werden diese Alkoholgruppen zweckmäßigerweise gegen Ozonolyse geschützt.

Das Verfahren eignet sich insbesondere für Verbindungen ausgewählt aus der Gruppe bestehend aus Manool, Sclareol, Larixol, Linalool, Nerolidol oder einem von den genannten Verbindungen abgeleiteten Derivat. Eine Derivatisierung der Verbindungen dient insbesondere der Einführung von Schutzgruppen für gegebenenfalls vorhandene nicht-allylische (nicht-alpha-ständige) Doppelbindungen, vorzugsweise durch deren selektive Epoxidierung.

Die Erfindung wird nachfolgend anhand von Ausführungsbeispielen näher erläutert.

### Allgemeine Angaben zur Reaktionsführung

Die Reaktionen wurden in herkömmlichen Laborapparaturen durchgeführt. In kleineren Ansätzen wurden die Reaktionsmischungen durch Trockeneisbäder bei der entsprechenden Temperatur gehalten. Bei größeren Ansätzen wurden Doppelmantelgefäße verwendet, durch die ein für den gewünschten Temperaturbereich geeignetes Kühlmedium gepumpt wurde.

In den Verbindungen vorhandene nicht-allylische Doppelbindungen wurden in der Regel vor der Ozonolyse durch Epoxidierung geschützt.

Soweit nicht anders angegeben, sind alle Angaben in % als Gewichts-%-Angaben zu verstehen. Mengenangaben in den Beispielen beziehen sich auf Massenverhältnisse.

Die folgenden Verbindungen mit einem diterpenischen Grundgerüst wurden in den Beispielen 1 bis 5 als Edukte eingesetzt: 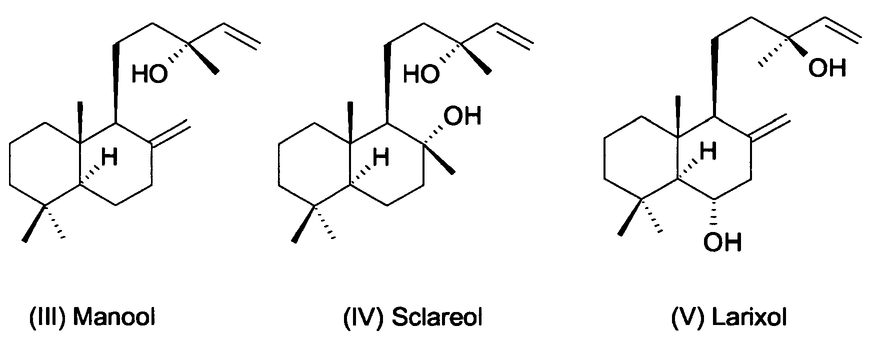 Die Verbindungen Manool **(III),** Sclareol **(IV)** und Larixol **(V)** sind durch Extraktion aus pflanzlichen Rohstoffen zugänglich und zeichnen sich durch eine tertiäre Alkoholfunktion mit einem allylischen Substituenten aus.

### Beispiel 1 - Umsetzung von Manool (III)

### a) Epoxidierung

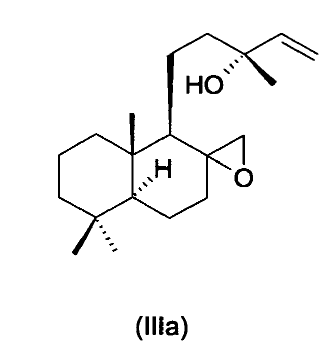

45 g / 0,16 mol Manool **(III)** wurden in 150 g Toluol vorgelegt und mit 0,32 g Tetrabutylammoniumhydrogensulfat versetzt. Nach Zusatz von 150 g Wasser wurden 110 g / 0,22 mol Magnesium-monoperoxyphthalat zugesetzt. Nach 4 h bei 40 °C wurde die organische Phase abgetrennt. Die abgetrennte organische Phase wurde nach Neutralisation durch gesättigte Na₂CO₃-Lösung mit Natriumsulfit-Lösung peroxidfrei gewaschen. Es fielen 39,8 g eines Produktes an, enthaltend die Verbindung **(IIIa)** in 80%iger Reinheit (GC-MS). Das Produkt wurde ohne weitere Aufarbeitung in der Ozonolyse eingesetzt.

### b) Ozonolyse

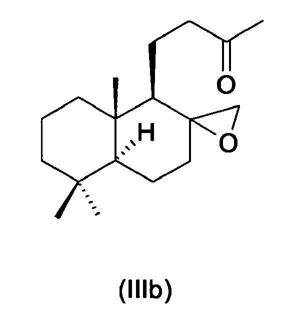

28,3 g / 0,08 mol der Verbindung **(IIIa)** wurden in der neunfachen Menge Toluol gelöst und auf -25°C abgekühlt. In die Reaktionsmischung wurden unter Rühren und Kühlen 2 Mol-Äquivalente Ozon mit konstanter Rate eingeleitet. Zur Reaktionsmischung wurden gleichzeitig 1,1 Mol-Äquivalente 5%iger wässriger NaOH-Lösung über die gesamte Dauer der Einleitung des Ozons zugetropft, wobei eine Zugaberate der Lösung konstant gehalten wurde. Ein Umsatz der Verbindung **(IIIa)** wurde mittels GC verfolgt. Nach vollständigem Umsatz wurde die Reaktionsmischung auf Raumtemperatur erwärmt, die wässrige Phase abgetrennt und die organische Phase mit wässriger gesättigter NaCl-Lösung neutral gewaschen.
Ausbeute an Verbindung **(IIIb):** 21,6 g (71 %)

### Beispiel 2 - Umsetzung von Larixol (V)

### a) Epoxidierung

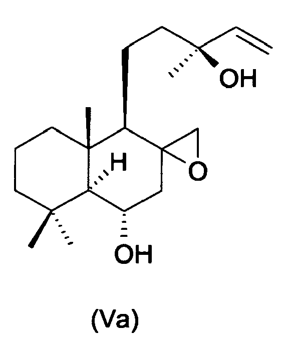

15 g / 0,06 mol Larixol **(V)** wurden in 150 g Toluol vorgelegt. Nach Zusatz von 150 g Wasser wurde 27,4 g Magnesium-monoperoxyphthalat zugesetzt. Nach 4 h Rühren bei 40 °C wurde die organische Phase abgetrennt. Diese wurde nach Neutralisation mit wässriger gesättigter NaCl-Lösung unter Verwendung von Natriumsulfitlösung peroxidfrei gewaschen. Es fielen 16,5 g eines Produktes an, enthaltend die Verbindung **(Va)** in 91%iger Reinheit (GC-MS). Das Produkt wurde ohne weitere Aufarbeitung in der Ozonolyse eingesetzt.

### b) Ozonolyse

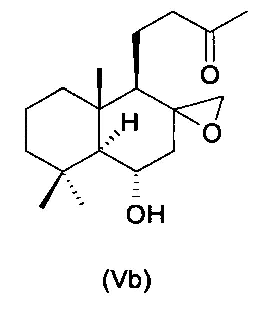

20,0 g / 0,06 mol der Verbindung **(Va)** wurden unter Rühren in 200 g CH₂Cl₂ gelöst und auf -40 °C abgekühlt. In die gekühlte Lösung wurden unter Rühren und Kühlen 2,5 Mol-Äquivalente Ozon mit konstanter Rate eingeleitet. Über die gesamte Dauer des Einleitens von Ozon wurde eine Lösung von 1,2 Mol-Äquivalenten NaOH (2,9 g / 0,07 mol), gelöst in 52 g Tetrahydrofuran und 52 g Wasser, mit konstanter Rate zugetropft. Nach Vertreiben des überschüssigen Ozons wurde der Reaktionsansatz auf Raumtemperatur erwärmt, dann mit wässriger gesättigter NaCl-Lösung neutralisiert und von gebildeten Peroxiden durch Waschen mit Natriumsulfit-Lösung befreit. Das Rohprodukt **(Vb)** fiel nach Abdestillieren des Lösungsmittels als helles bis farbloses Öl an.

Die Ausbeute beträgt 70% (GC-MS) berechnet auf Verbindung **(V)**.

### Beispiel 3 - Ozonolyse von Sclareol (IV)

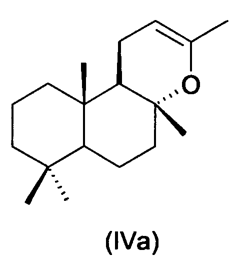

30,8 g / 0,1 mol Sclareol (IV) wurden in der 4,5-fachen Menge Methanol/CH₂Cl₂ (1:1 (m/m)) gelöst und die Reaktionsmischung auf -20 °C abgekühlt. In die Reaktionsmischung wurden anschließend unter Rühren und Kühlen 2 Mol-Äquivalente Ozon mit konstanter Rate eingeleitet. Während der Zugabe des Ozons wurden in die Reaktionsmischung 1,1 Mol-Äquivalente NaOH, bezogen auf Sclareol und gelöst in der fünffachen Menge Wasser und Tetrahydrofuran, mit konstanter Rate zugetropft. Die Reaktionsmischung wurde nach Beendigung der Reaktion auf Raumtemperatur erwärmt und die organische Phase abgetrennt. Nach Neutralisation mit wässriger gesättigter NaCl-Lösung und Vernichtung entstandener Peroxide mit Natriumsulfit-Lösung wurde das Lösungsmittel abdestilliert und das Reaktionsprodukt Sclareoloxid **(IVa)** mit einer Ausbeute von 97 % erhalten (GC-MS).

### Beispiel 4 - Ozonolyse von Sclareol (IV) unter Verwendung verschiedener Basen

162 g / 0,5 mol Sclareol **(IV)** wurden in der neunfachen Menge Toluol gelöst und die Reaktionsmischung auf -5 °C abgekühlt. In die Reaktionsmischung wurden unter Rühren und Kühlen anschließend 2 Mol-Äquivalente Ozon mit konstanter Rate eingeleitet. Während der Zugabe des Ozons wurden in die Reaktionsmischung 1,5 Mol-Äquivalente der in Tab.1 genannten Basen, bezogen auf Sclareol **(IV)** und gelöst in der fünffachen Menge Wasser und Tetrahydrofuran, mit konstanter Rate zugetropft. Die Reaktionsmischung wurde nach Beendigung der Reaktion auf Raumtemperatur erwärmt und die organische Phase abgetrennt. Nach Neutralisation der organischen Phase mit wässriger gesättigter NaCl-Lösung, Wäsche mit einer Natriumsulfit-Lösung und Abdestillieren des Lösungsmittels wurde das Reaktionsprodukt als gelber Feststoff erhalten und der Gehalt an Sclareol **(IV)** und Sclareoloxid **(IVa)** per GC-MS bestimmt (siehe Tab.1).

Die höchste Selektivität beim Umsatz von Sclareol **(IV)** zu Sclareoloxid **(IVa)** wurde mit einer Lösung von KOH erreicht.

**Tab. 1**

| ***Base*** | ***Umsatz _{Sclareol} [%]*** | ***Sclareol (IV) [%]*** | ***Sclareoloxid (IVa) [%]*** |
|---|---|---|---|
| ***KOH*** | ***99,5*** | ***0,5*** | ***96,4*** |
| ***NaHCO₃*** | ***96,5*** | ***3,5*** | ***79,4*** |
| ***Na₂CO₃*** | ***99,5*** | ***0,5*** | ***62,9*** |
| ***CaCO₃*** | ***98,1*** | ***1,9*** | ***78,0*** |

### Beispiel 5 - Ozonolyse von Manool (III)

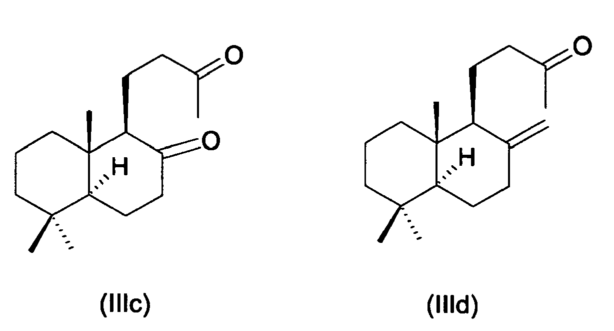

82,0 g / 0,20 mol Manool **(III)** (70%iger Naturstoff) wurden in 250 g Toluol vorgelegt und auf -5 °C abgekühlt. In die Reaktionsmischung wurden unter Rühren und Kühlen anschließend 2,5 Mol-Äquivalente Ozon mit konstanter Rate eingeleitet. Während der gesamten Reaktionszeit wurde zur Reaktionsmischung eine Lösung aus 12 g / 0,3 mol NaOH und Wasser (Massenverhältnis 1:9) mit konstanter Rate zugetropft. Nach Beendigung der Einleitung des Ozons wurde die Reaktionsmischung auf Raumtemperatur erwärmt und die organische Phase abgetrennt. Die organische Phase wurde durch mehrfaches Waschen mit Wasser neutralisiert. Nach dem Einengen der organischen Phase wurde das Rohprodukt als farbloses Öl erhalten.

| | | |
|---|---|---|
| Zusammensetzung (GC-MS): | Verbindung **(IIIc)** | 66% |
| | Verbindung **(IIId)** | 33% |

### Beispiel 6 - Umsetzung von Linalcool (VI)

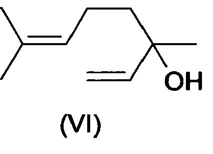

### a) Epoxidierung

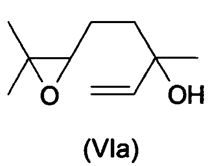

7,7 g Linalool (VI) wurden in 100 g Toluol gelöst und mit 0,1 g Tetrabutylammoniumhydrogensulfat versetzt und analog den Vorschriften unter Beispiel 1, a) Epoxidierung umgesetzt und aufgearbeitet.

Rohausbeute: 7,4 g, davon Gehalt an der Verbindung **(Vla)** 88%

### b) Ozonolyse

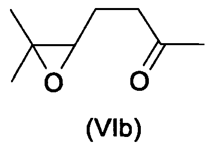

5,0 g der Verbindung **(VIa)** wurden in 100 g CH₂Cl₂ gelöst und analog den Vorschriften unter Beispiel 1, b) Ozonolyse umgesetzt. Nach Aufarbeitung der Reaktionsmischung (Neutralwäsche mit NaCl-Lösung, Natriumsulfitwäsche) wurden 2,15 g an 5,6-Epoxy-6-Methyl-heptan-2-on **(VIb)** erhalten (Ausbeute: 49% (GC-MS)).

### Beispiel 7 - Umsetzung von Nerolidol (VII)

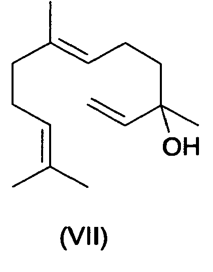

### a) Epoxidierung

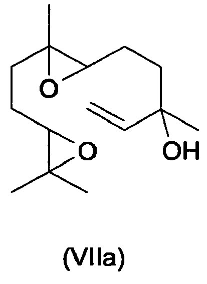

22,0 g Nerolidol **(VII)** wurden in 100 g Toluol gelöst und mit 0,3 g Tetrabutylammoniumhydrogensulfat versetzt und analog den Vorschriften unter Beispiel 1, a) Epoxidierung umgesetzt und aufgearbeitet.

Es wurden 13,9 g der Verbindung **(VIIa)** erhalten.

### b) Ozonolyse

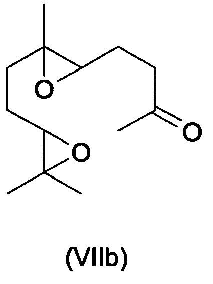

Es wurden 7,5 g der Verbindung (**VIIa)** gelöst in 100 g CH₂Cl₂ vorgelegt. Anschließend wurden unter Rühren bei -40 °C 2,5 Mol-Äquivalente Ozon mit konstanter Rate in die Reaktionsmischung eingeleitet. Während der gesamten Reaktion wurden 1,1 Mol-Äquivalente NaOH, gelöst in der neunfachen Menge Wasser/Tetrahydrofuran (1/1, m/m), mit konstanter Rate eingeleitet. Die Reaktionsmischung wurde nach Beendigung der Ozonolyse auf Raumtemperatur erwärmt und die organische Phase abgetrennt. Nach Neutralisation der organischen Phase mit Wasser und destillativer Entfernung des Lösemittels wurde die Verbindung **(VIIb)** als farbloses Öl erhalten.

Ausbeute: 4,33 g (43,70%)

## Patentansprüche

1. Verfahren zur Darstellung eines Ketons aus einem tertiären Alkohol mit einer alpha-ständigen Doppelbindung, umfassend die Schritte:
a) Bereitstellen des Alkohols,
b) Behandeln des Alkohols mit Ozon in Anwesenheit einer anorganischen Base.

2. Verfahren nach Anspruch 1, **dadurch gekennzeichnet, dass** in Schritt b) der eingesetzte Alkohol mit 1-3 Moläquivalenten Ozon je zu behandelnder Alkohol-Gruppe behandelt wird, und vorzugsweise mit 1-2 Moläquivalenten Ozon je zu behandelnder Alkohol-Gruppe behandelt wird.

3. Verfahren nach einem der vorherigen Ansprüche, **dadurch gekennzeichnet, dass** in Schritt b) die Base so zugesetzt wird, dass ihre Äquivalentkonzentration bezogen auf die zu behandelnde Alkoholgruppe 1 bis 3, vorzugsweise 1 bis 2 beträgt.

4. Verfahren nach einem der vorherigen Ansprüche, **dadurch gekennzeichnet, dass** die Base in Schritt b) ausgewählt ist aus der Gruppe bestehend aus NaOH, KOH, LiOH, NaHCO₃, Na₂CO₃, CaCO₃ oder Mischungen zweier oder mehrerer dieser Basen.

5. Verfahren nach einem der vorherigen Ansprüche, **dadurch gekennzeichnet, dass** in Schritt a) der Alkohol in einem organischen Lösungsmittel bereitgestellt wird und in Schritt b) die Base in einem wässrigen Lösungsmittel eingesetzt wird.

6. Verfahren nach einem der vorherigen Ansprüche, **dadurch gekennzeichnet, dass** in Schritt b) die Reaktionstemperatur im Bereich von -78 °C bis +30 °C beträgt.

7. Verfahren nach einem der vorherigen Ansprüche, **dadurch gekennzeichnet, dass** der Alkohol die allgemeine Formel (la) besitzt: 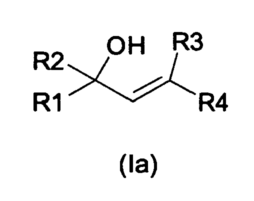
wobei R1 und R2, unabhängig voneinander, einen organischen Rest bedeuten und beide Reste R1 und R2 gemeinsam einen Ring bilden können, und wobei ferner
R3 und R4, unabhängig voneinander, Wasserstoff oder substituiertes oder nicht substituiertes Alkyl, Alkenyl, Cycloalkyl oder Aryl bedeuten können und beide Reste gemeinsam einen Ring bilden können und/oder einer oder beide Reste R3 und R4 mit einem oder beiden Resten R1 und/oder R2 gemeinsam einen Ring bilden können.

8. Verfahren nach einem der vorherigen Ansprüche, **dadurch gekennzeichnet, dass** der eingesetzte Alkohol ausgewählt ist aus der Gruppe bestehend aus

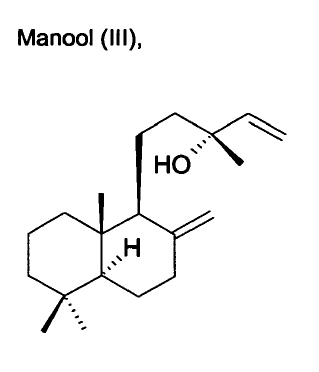

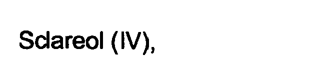

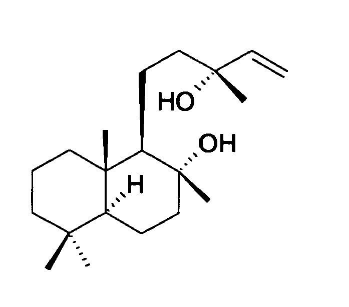

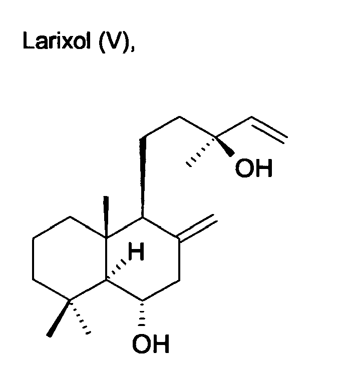

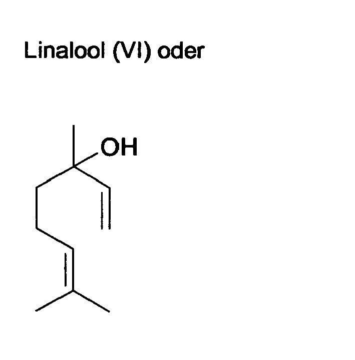

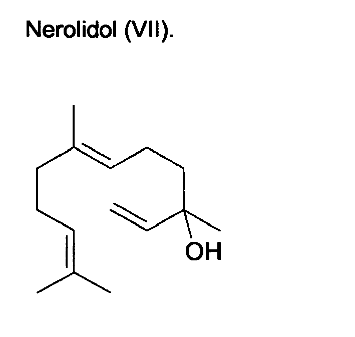

## Claims

1. Process for the preparation of a ketone from a tertiary alcohol having a double bond in the alpha position, comprising the steps:
a) provision of the alcohol,
b) treatment of the alcohol with ozone in the presence of an inorganic base.

2. Process according to claim 1, **characterized in that** in step b) the alcohol employed is treated with 1-3 molar equivalents of ozone per alcohol group to be treated, and preferably is treated with 1-2 molar equivalents of ozone per alcohol group to be treated.

3. Process according to one of the preceding claims, **characterized in that** in step b) the base is added such that its equivalent concentration is 1 to 3, preferably 1 to 2, based on the alcohol group to be treated.

4. Process according to one of the preceding claims, **characterized in that** the base in step b) is chosen from the group consisting of NaOH, KOH, LiOH, NaHCO₃, Na₂CO₃, CaCO₃ or mixtures of two or more of these bases.

5. Process according to one of the preceding claims, **characterized in that** in step a) the alcohol is provided in an organic solvent and in step b) the base is employed in an aqueous solvent.

6. Process according to one of the preceding claims, **characterized in that** in step b) the reaction temperature is in the range of from -78 °C to +30 °C.

7. Process according to one of the preceding claims, **characterized in that** the alcohol has the general formula (la): 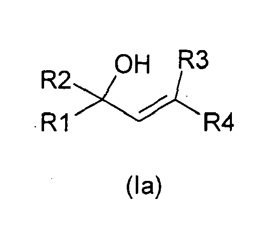
wherein R1 and R2 independently of one another denote an organic radical and the two radicals R1 and R2 together can form a ring, and wherein furthermore
R3 and R4 independently of one another can denote hydrogen or substituted or unsubstituted alkyl, alkenyl, cycloalkyl or aryl and the two radicals together can form a ring and/or one or both of the radicals R3 and R4 can form a ring together with one or both of the radicals R1 and/or R2.

8. Process according to one of the preceding claims, **characterized in that** the alcohol employed is chosen from the group consisting of

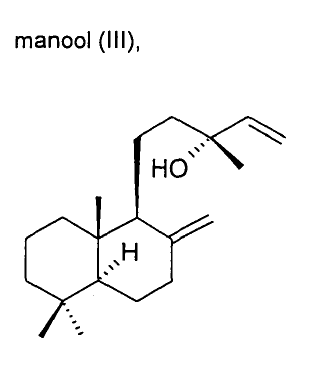

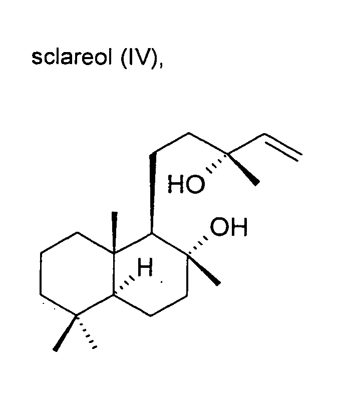

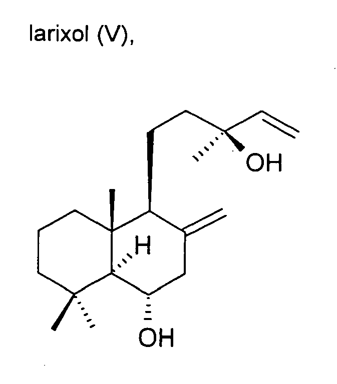

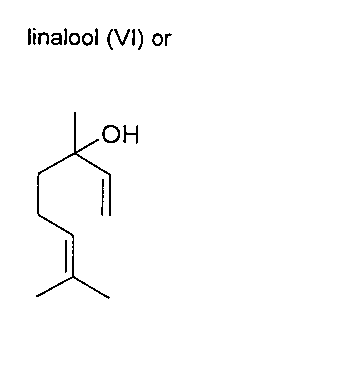

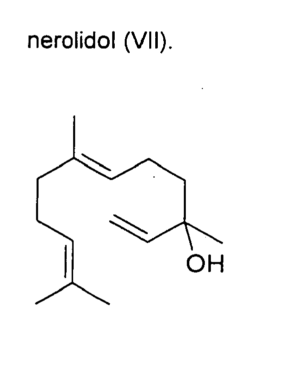

## Revendications

1. Procédé pour la préparation d'une cétone à partir d'un alcool tertiaire avec une double liaison en positon alpha comprenant les étapes :
a) de préparation de l'alcool,
b) de traitement de l'alcool avec de l'ozone en présence d'une base inorganique.

2. Procédé selon la revendication 1, **caractérisé en ce que** l'alcool utilisé avec 1-3 équivalents molaires d'ozone par rapport à chaque fois par groupe alcool à traiter est traité dans l'étape b) et est de préférence traité avec 1-2 équivalents molaires d'ozone par rapport à chaque groupe alcool à traiter.

3. Procédé selon l'une quelconque des revendications précédentes, **caractérisé en ce que** l'on ajoute dans l'étape b) la base, de telle sorte que sa concentration en équivalent rapportée au groupe alcool à traiter est de 1 à 3, de préférence de 1 à 2.

4. Procédé selon l'une quelconque des revendications précédentes, **caractérisé en ce que** la base dans l'étape b) est choisie parmi NaOH, KOH, LiOH, NaHCO₃, Na₂CO₃, CaCO₃ ou des mélanges de deux ou plusieurs de ces bases.

5. Procédé selon l'une quelconque des revendications précédentes, **caractérisé en ce que** dans l'étape a) l'alcool est mis à disposition dans un solvant organique et **en ce que** la base est utilisée dans l'étape b) dans un solvant aqueux.

6. Procédé selon l'une quelconque des revendications précédentes, **caractérisé en ce que** la température de la réaction se trouve dans l'étape b) dans un domaine de -78°C à +30°C.

7. Procédé selon l'une quelconque des revendications précédentes, **caractérisé en ce que** l'alcool présente la formule générale (Ia) : 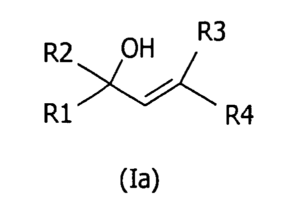
dans laquelle R1 et R2 représentent indépendamment l'un de l'autre un reste organique et les deux restes R1 et R2 peuvent former ensemble un cycle, et dans laquelle de plus
R3 et R4 peuvent représenter indépendamment l'un de l'autre un atome d'hydrogène ou un groupe alkyle, alcényle, cycloalkyle ou aryle substitué ou non substitué et les deux restes peuvent former ensemble un cycle et/ou un des deux ou les deux restes R3 et R4 peuvent former ensemble un cycle avec un des deux ou les deux restes R1 et/ou R2.

8. Procédé selon l'une quelconque des revendications précédentes, **caractérisé en ce que** l'on choisit l'alcool utilisé parmi
